# EUROPEAN PATENT APPLICATION

(11) **EP 4 548 911 A1**
(43) Date of publication of application: **07.05.2025**
(21) Application number: 24210946.0
(22) Date of filing: 05.11.2024
(51) Int. Cl.: A61K 9/00, A61K 9/20, A61K 45/06, A61K 31/00

(54) **PHARMACEUTICAL COMPOSITIONS**

(30) Priority: 06.11.2023 IN 202341075527
(71) Applicant: Extrovis AG, 6340 Baar (CH); Athyna Pharma LLC, Kendall Park, NJ 08824 (US)
(72) Inventor: Kowkuntla, Kirankumar, Kendall Park (US)
(74) Representative: Nederlandsch Octrooibureau

(57) **Abstract**

The present disclosure relates to a stable, fast-disintegrating pharmaceutical tablet for buccal or sublingual administration, containing buprenorphine or its pharmaceutically acceptable salt, and naloxone or its pharmaceutically acceptable salt, providing enhanced ease of administration. The stable pharmaceutical tablet disintegrates in a patient's oral cavity, under the tongue, and helps in sublingual absorption of the buprenorphine or its pharmaceutically acceptable salt.

## Description

The present disclosure relates to a stable, fast-disintegrating pharmaceutical tablet designed for buccal or sublingual administration, containing buprenorphine or its pharmaceutically acceptable salt, and naloxone or its pharmaceutically acceptable salt, providing enhanced ease of administration. The stable pharmaceutical tablet disintegrates in a patient's oral cavity, under the tongue, and helps in sublingual absorption of the buprenorphine or its pharmaceutically acceptable salt.

### Background of the invention

Opioids are a class of medication used in the management and treatment of pain. Opioids act both presynaptic ally and post-synaptically to produce an analgesic effect. However, opioid analgesics are sometimes the subject of abuse. Opioid use disorder (OUD) is a treatable chronic disorder with episodes of remission and recurrence characterized by loss of control of opioid use, compulsive use, and continued use despite harms. OUD affects over 26 million individuals worldwide. There are currently three World Health Organization recommended and Food and Drug Administration (FDA) approved medication treatments for opioid use disorder : the full opioid agonist methadone, the opioid partial-agonist buprenorphine, and the opioid-receptor antagonist naltrexone. Comparisons of buprenorphine to full opioid agonists such as methadone and hydromorphone suggest that sublingual buprenorphine produces typical opioid agonist effects which are limited by a ceiling effect. Buprenorphine is a partial agonist at the mu-opioid receptor and an antagonist at the kappa- opioid receptor. Naloxone is an opioid antagonist and produces opioid withdrawal signs and symptoms in individuals physically dependent on full opioid agonists when administered parenterally. The combination of buprenorphine and naloxone, therefore, is helpful in preventing or reducing abuse of the drug.

Buprenorphine (commercially available as Subutex^{®} in the United States of America), as a sublingual tablet formulation, has been available as an opioid-addiction treatment medication since 1996 in France, and in the United States since 2003.

A sublingual tablet formulation containing a combination of buprenorphine and naloxone (commercially available as Suboxone^{®} in the United States of America) in a 4:1 ratio (i.e., buprenorphine 2 mg/naloxone 0.5 mg, or buprenorphine 8 mg/naloxone 2 mg) has been in the market in USA since October 2002, to mitigate the potential diversion and abuse of buprenorphine. The USFDA approved label for this product advises the patient to place all the tablets under the tongue at the same time (for doses requiring more than two tablets), or to place two tablets under the tongue at a time (if they cannot fit more than two tablets comfortably) for doses requiring more than two tablets. To maximize the bioavailability of the drug, the tablets are required to be held under the tongue until they dissolve. Swallowing the tablets has been reported to reduce their bioavailability. As can be envisaged, patient compliance is reduced due to this dosage regimen requiring use of 2 or more tablets, resulting in poor opioid maintenance treatment outcomes.

Suboxone^{®} sublingual film (12/3 mg, 2/0.5 mg, 8/2 mg & 4/1 mg) and Cassipa^{®} (16/4 mg) sublingual film are available in alternate strengths, including higher strengths. Suboxone^{®} sublingual film label states that `Patients can be switched between Buprenorphine and Naloxone or Buprenorphine only sublingual tablets, and Suboxone^{®} sublingual film should be started on the same dosage of the previously administered product. However, dosage adjustments may be necessary when switching between Buprenorphine products. Not all strengths and combinations of the Suboxone sublingual films are bioequivalent to Suboxone^{®} sublingual tablets. This also makes it difficult for physicians to switch patients from sublingual tablets to sublingual films and vice versa. Cassipa label also states that "Not all strengths and combinations of buprenorphine hydrochloride and naloxone hydrochloride sublingual films are bioequivalent to buprenorphine hydrochloride and naloxone hydrochloride sublingual tablets as observed in pharmacokinetic studies. Therefore, systemic exposures of buprenorphine and naloxone may be different when patients are switched from tablets to film or vice-versa. Patients should be monitored for symptoms related to overdosing or under-dosing"

The differences in Suboxone Film and Cassipa film compared to currently marketed Buprenorphine and Naloxone Sublingual Tablets pose a problem of dosing appropriate amount of drugs for safe and effective treatment.

Ahmadi et al ("Rapid effect of a single-dose buprenorphine on reduction of opioid craving and suicidal ideation: A randomized, double blind, placebo-controlled study"; Tzu Chi Medical Journal 2020; 32(1): 58-64) assessed the influence of a single, physicianadministered dose of buprenorphine on withdrawal craving and suicidal ideation in opioid-dependent patients. A single high dose of 16 mg or 32 mg buprenorphine was reported to reduce opioid craving.

United States Patent 8,470,361 covers a particulate transmucosal pharmaceutical composition in the form of a tablet comprising an opioid analgesic in combination with an opioid antagonist presented in particulate form upon the surfaces of carrier particles.

International patent application WO2008104737A1 discloses a composition for the treatment of pain in human patients wherein said composition comprises buprenorphine to naloxone in a ratio by weight of from 2.1:1 to 8:1, and the preferred amount of buprenorphine per dosage unit is from 10 µg to 8 mg.

There remains a need for new strengths of Buprenorphine/Naloxone in the form of sublingual tablets that would greatly improve patient compliance and give doctors a simpler and more straightforward way to prescribe Buprenorphine/Naloxone at doses that are effective in individual cases. Further, there is also a need to provide a dosage form containing buprenorphine and naloxone that releases both the active ingredients at a rate such that the desired bioavailability is achieved, thereby providing the desired efficacy. Designing the new strengths to be bioequivalent to the existing strengths (at equal dose), for example, ONE tablet of the new strength of Buprenorphine/Naloxone Tablets, 16/4 mg would be bioequivalent to TWO tablets of Buprenorphine/Naloxone Tablets, 8/2 mg, would also ensure that the healthcare providers may seamlessly switch patients from current strengths to the new strengths, which would be equal dose and without the need for titration.

### Summary of the invention

The present disclosure provides a stable pharmaceutical tablet for buccal or sublingual administration comprising from about 1mg to about 24mg of buprenorphine or pharmaceutically acceptable salt thereof, and from about 0.5mg to about 6mg of naloxone or pharmaceutically acceptable salt thereof.

The stable pharmaceutical tablet of the present disclosure contains buprenorphine or its pharmaceutically acceptable salt, and naloxone or its pharmaceutically acceptable salt, wherein the tablet is for sublingual or buccal administration, having an in vitro disintegration time of less than 120 seconds, when tested by the disintegration test defined in the United States Pharmacopoeia (USP).

The stable pharmaceutical compositions of the present disclosure contain buprenorphine or its pharmaceutically acceptable salt, and naloxone or its pharmaceutically acceptable salt, wherein the composition is in the form of a sublingual or buccal tablet having in-vivo disintegration time of less than 20 minutes when the tablet is administered without any pre-hydration of the mouth with water. In-vivo disintegration is measured as time taken for the tablets to fully disintegrate under the tongue, or in the mouth, without disturbing, moving or chewing the tablets.

The stable pharmaceutical compositions of the present disclosure contain buprenorphine or its pharmaceutically acceptable salt, and naloxone or its pharmaceutically acceptable salt, wherein the composition is in the form of a sublingual or buccal tablet that has an in-vivo disintegration time of less than 10 minutes when the tablet is administered after pre-hydration of the mouth with water.

The pharmaceutical tablet of the present disclosure contains buprenorphine or its pharmaceutically acceptable salt, and naloxone or its pharmaceutically acceptable salt, wherein the composition is in the form of a sublingual or buccal tablet having hardness of about 2kP to about 10kP. The tablets of the present disclosure have friability of less than 1%.

The present disclosure provides a stable pharmaceutical tablet for buccal or sublingual administration comprising from about 1mg to about 24mg buprenorphine in the form of its base or pharmaceutically acceptable salt, and from about 0.5 mg to about 6mg naloxone in the form of its base or pharmaceutically acceptable salt, wherein
- the tablet has an in vitro disintegration time of less than 120 seconds when tested by the disintegration test defined in the United States Pharmacopoeia (USP),
- hardness of 2-10kP,
- friability of less than 1%,
- in vivo disintegration time of less than 20 minutes, and
releases more than 85% of the buprenorphine or its pharmaceutically acceptable salt in less than 5 minutes, and all of the naloxone or its pharmaceutically acceptable salt in less than 20 minutes, when tested in vitro in a dissolution apparatus defined in the United States Pharmacopoeia (USP).

The pharmaceutical tablet of the present disclosure comprises pharmaceutically acceptable excipients selected from one or more of disintegrating agent, filler, lubricant, diluent, binder, pH adjusting agent, sweetening agent, flavoring agent and combinations thereof.

In a preferred embodiment, the pharmaceutical tablet of the present disclosure may comprise the buprenorphine or pharmaceutically acceptable salt thereof in an amount equivalent to about 16 mg, and naloxone or pharmaceutically acceptable salt thereof in an amount equivalent to about 4 mg.

In another preferred embodiment, the pharmaceutical tablet of the present disclosure may comprise the buprenorphine or pharmaceutically acceptable salt thereof in an amount equivalent to about 12 mg, and naloxone or pharmaceutically acceptable salt thereof in an amount equivalent to about 3 mg.

In yet another preferred embodiment, the pharmaceutical tablet of the present disclosure may comprise the buprenorphine or pharmaceutically acceptable salt thereof in an amount equivalent to about 4 mg, and naloxone or pharmaceutically acceptable salt thereof in an amount equivalent to about 1 mg.

In still another preferred embodiment, the pharmaceutical tablet of the present disclosure may comprise the buprenorphine or pharmaceutically acceptable salt thereof in an amount equivalent to about 24 mg, and naloxone or pharmaceutically acceptable salt thereof in an amount equivalent to about 6 mg.

In some embodiments, the pharmaceutical tablet of the present disclosure comprises a score mark or separation mark to guide a user in breaking the tablet along the designated area or region indicated by said score or separation mark. The scored tablet, when broken along the score line, provides equal parts that each contain equal amounts of buprenorphine and naloxone. In some other embodiments, the pharmaceutical tablet does not include any separation or score mark.

In a preferred embodiment, the pharmaceutical tablet of the present disclosure is a stable pharmaceutical tablet for buccal or sublingual administration comprising about 1mg to about 24mg of buprenorphine in the form of its base or pharmaceutically acceptable salt, and about 0.5mg to about 6mg of naloxone in the form of its base or pharmaceutically acceptable salt, such that upon storage at accelerated storage conditions (40⁰ C/75 % relative humidity) for at least one month, the tablet contains about 0.1% or less of 3-O-allyl naloxone impurity and about 0.1% or less of Buprenorphine Impurity G. In a preferred embodiment, the stable pharmaceutical tablet comprises a total amount of naloxone related degradants of about 0.2% or less, and a total amount of buprenorphine related degradants of about 0.2% or less.

### Detailed description of the invention

Buprenorphine or [5α,7α(S)]-17-(Cyclopropylmethyl)-α-(1,1-dimethylethyl)-4,5- epoxy-18,19- dihydo-3-hydroxy-6-methoxy-α-methyl-6,14-ethenomorphinan-7-methanol is a partial agonist at the µ-opioid receptor and an antagonist at the K-opioid receptor. It has high binding affinity at both receptors and competes with other agonists, such as methadone, heroin (diamorphine) and morphine, at the µ-opioid receptor. Buprenorphine is a Schedule III opioid analgesic with unique pharmacodynamic and pharmacokinetic properties that may be preferable to those of Schedule II full µ-opioid receptor agonists. Partial agonism at the µ-opioid receptor does not provide partial analgesia, but rather analgesia equivalent to that of full µ-opioid receptor agonists. Opioid agonist effects of buprenorphine are less than the maximal effects of other, full opioid agonists, such as morphine, and are limited by a "ceiling effect". The drug thus produces a lower degree of physical dependence than other opioid agonists, such as heroin, morphine or methadone, and is therefore, particularly useful in substitution therapy.

As used herein, unless indicated otherwise, the term "buprenorphine", includes any pharmaceutically acceptable form of buprenorphine, including, but not limited to, salts, esters, and prodrugs thereof. Basic chemical structure of buprenorphine can be presented as below -

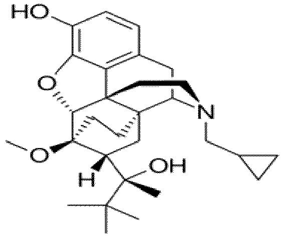

Because buprenorphine alone, as a (partial) µ-opioid-receptor agonist, is subject to abuse, the combination treatment with naloxone was intended to minimize the abuse and misuse of the compound.

Naloxone or N-Allylnoroxymorphone, chemically known as 17-Allyl-4,5α-epoxy-3,14-dihydroxymorphinan-6-one, is a non-selective, competitive and short-acting opioid receptor antagonist that has a long clinical history of successful use, and is presently considered a safe drug over a wide dose range (up to 10 mg). Naloxone was patented in 1961 and approved for opioid overdose in the United States in 1971. In opioid-dependent patients, naloxone is used in the treatment of opioid-overdose-induced respiratory depression, in (ultra)rapid detoxification and in combination with buprenorphine for maintenance therapy (to prevent intravenous abuse). Basic chemical structure of naloxone can be presented as below -

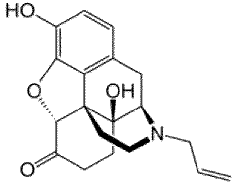

Each stable pharmaceutical tablet for buccal or sublingual administration of the present disclosure contains about 1mg to about 24mg of buprenorphine or pharmaceutically acceptable salt thereof, and about 0.5mg to about 6mg naloxone e or pharmaceutically acceptable salt thereof.

The maintenance dose of currently approved buprenorphine hydrochloride/naloxone hydrochloride Suboxone^{®} sublingual tablets is generally in the range of 4 mg/1 mg buprenorphine/naloxone to 24 mg/6 mg buprenorphine/naloxone per day, depending on the individual patient. The recommended target dosage of buprenorphine and naloxone sublingual tablets is 16mg/4 mg buprenorphine/naloxone per day as a single daily dose. There are only two approved strengths of buprenorphine/naloxone (8/2 mg and 2/0.5 mg) sublingual tablets at the moment, and multiple tablets must be administered at once to achieve maintenance treatment. The present disclosure provides new strengths of buprenorphine/naloxone sublingual tablets (24/6 mg, 16/4 mg, 12/3 mg, 4/1 mg) that are expected to significantly increase patient compliance and provide ease of administration.

Furthermore, the tablets may be scored to increase dosage flexibility. Typically, the score or score mark is a bisecting score, which may provide a suggestion that the scored tablet is divisible into two equal portions. Other patterns such as trisection, including trisection in the presence of bisection, which may suggest divisibility into at least three equal portions, and quadrisection, which may suggest divisibility into four equal sections, may also be provided. The scored tablets allow the physician the ability to design the dose for individual patient, especially in cases where the dose is adjusted in increments/decrements of 2 mg/0.5 mg or 4 mg/1 mg buprenorphine/naloxone to a level that holds the patient in treatment and suppresses opioid withdrawal signs and symptoms. The scored tablets of the present disclosure may also be labeled to instruct the user to swallow the tablet whole.

In the present disclosure, a stable pharmaceutical tablet containing new strengths of buprenorphine and naloxone, for buccal or sublingual administration, is designed to be administered as a 'single' unit (as a whole tablet or split tablet) to achieve various induction, titration, and maintenance doses as indicated on the product label. These new strengths are intended to be bioequivalent to the Suboxone^{®} tablets (currently unavailable in the market), i.e., they are intended to have the same bioavailability, safety and efficacy as the Suboxone^{®} tablets.

The stable pharmaceutical tablet of the present disclosure, which is for suitable for buccal or sublingual administration comprises buprenorphine and naloxone as the active ingredients, and excipients selected from one or more of disintegrating agent, filler, lubricant, diluent, binder, pH adjusting agent, sweetening agent, flavouring agent, coloring agent and combinations thereof.

The stable pharmaceutical tablet composition of the present disclosure comprises an effective amount of buprenorphine or salt thereof in the range of about 1%w/w to about 15%w/w of the total composition. In preferred embodiments, buprenorphine is present in the range of about 1%w/w to about 10%w/w, preferably in the range of about 1%w/w to about 5%w/w of the total composition. The stable pharmaceutical tablet composition comprises buprenorphine in an amount ranging from about 1mg to about 24mg, preferably in the range of about 1mg, 2mg, 3mg, 4mg, 5mg, 6mg, 7mg, 8mg, 9mg, 10mg, 11mg, 12mg, 13mg, 14mg,15mg, 16mg, 17mg, 18mg, 19mg, 20mg, 21mg, 22mg, 23mg or 24mg.

The stable pharmaceutical tablet composition of the present disclosure comprises an effective amount of naloxone or salt thereof in the range of about 1%w/w to about 5%w/w of the total composition. In preferred embodiments, naloxone is present in the range of about 1%w/w to about 3%w/w, preferably in the range of about 1%w/w to about 2%w/w of the total composition. The stable pharmaceutical tablet composition comprises naloxone in an amount ranging from about 0.5mg to about 5mg, preferably in the range of about 0.5mg, 1mg, 2mg, 3mg, 4mg, 5mg or 6mg.

Disintegrants are added to a composition to promote the drug release of the active ingredient(s) therefrom. They do this by increased water wicking into the composition, and by promoting de-aggregation of the compacted or granulated particles. There are two classes of disintegrants: traditional disintegrants, such as starch, and super disintegrants, which include agents such as, but not limited to, croscarmellose sodium, crosslinked polyvinylpyrrolidone (PVP), crosslinked starch, sodium starch glycolate, crosslinked alginic acid, soy polysaccharides, and the like. The disintegrant is typically present in the range of about 1% w/w to about 20% w/w of the total composition. Preferably, the disintegrant is present in the range of about 1% w/w to about 19% w/w, more preferably in the range of about 1% w/w to about 18% w/w, still more preferably in the range of about 1% w/w to about 17% w/w, more preferably in the range of about 1% w/w to 16% w/w, more preferably in the range of about 1% w/w to about 15% w/w, more preferably in the range of about 1% w/w to about 14% w/w, more preferably in the range of about 1% w/w to about 13% w/w, more preferably in the range of about 1% w/w to about 12%w/w, more preferably in the range of about 1% w/w to about 10% w/w of the total composition.

Fillers that may be used in the pharmaceutical compositions of the present disclosure are selected from lactose monohydrate, mannitol, sorbitol, cellulose, calcium phosphate, starch, sugar, cellulose, modified cellulose, sodium carboxymethyl cellulose, ethyl cellulose hydroxymethyl cellulose, hydroxypropylcellulose, cellulose acetate, microcrystalline cellulose, dibasic calcium phosphate, sucrose, lactose, corn starch, potato starch, or any combination thereof. In one embodiment, the filler is lactose monohydrate and is present in the tablet in an amount ranging from about 10% w/w to about 90 % w/w of the total composition. In one embodiment, the filler is a mixture of microcrystalline cellulose (MCC) and lactose monohydrate, and the mixture is present in the tablet in an amount ranging from about 40%w/w to about 90%w/w of the total composition.

The stable pharmaceutical tablet composition generally also includes a lubricant. Examples of lubricants include, but are not limited to, magnesium stearate, calcium stearate, sodium stearyl fumarate, stearic acid, glyceryl behenate, polyethylene glycol, ethylene oxide polymers, sodium lauryl sulphate, magnesium lauryl stearate, hydrogenated vegetable oil and mixtures thereof. Preferred lubricants include calcium stearate, magnesium stearate and sodium stearyl fumarate. Most preferred as the lubricant is magnesium stearate. Lubricants generally comprise from about 0.5%w/w to about 5%w/w of the total composition. The amount of lubricant employed is generally from about 1 to about 2%w/w, preferably about 0.5 to 2% w/w of the total composition.

Diluents may be added to aid compression, and usually include one or more of microcrystalline cellulose, lactose, mannitol, calcium salts such as calcium phosphate dibasic, calcium sulphate and calcium carbonate, and sugars such as lactose, sucrose, dextrose and maltodextrin. Preferred diluents are microcrystalline cellulose, lactose and mannitol. Spraydried forms of lactose and mannitol are particularly suitable forms of those compounds for direct compression or dry granulation techniques. In one embodiment, the filler is mannitol and is present in the tablet in an amount ranging from about 10% w/w to about 90 % w/w, more preferably in the range of about 10% w/w to about 80% w/w, more preferably in the range of about 10% w/w to about 70% w/w of the total composition.

Binders are excipients which contribute to particle adhesion. Examples of binders that may be used in the pharmaceutical compositions of the present disclosure include, but are not limited to, carboxymethylcellulose, hydroxypropyl methylcellulose, hydroxypropyl cellulose, hydroxyethylcellulose, ethylcellulose, microcrystalline cellulose, lactose, sucrose, dextrose, glucose, maltodextrin, mannitol, xylitol, polymethacrylates, polyvinylpyrrolidone, sorbitol, pregelatinized starch, alginic acids and salts thereof such as sodium alginate, magnesium aluminum silicate, polyethylene glycol, carrageenan and the like, and mixtures thereof. Generally, the amount of binder can vary widely, for example, from about 1% to about 10% w/w of the total composition, more preferably in the range of about 1% w/w to about 9% w/w, more preferably in the range of about 1% w/w to about 8% w/w, more preferably in the range of about 1% w/w to about 7% w/w, more preferably in the range of about 1% w/w to about 6% w/w, more preferably in the range of about 1% w/w to about 5% w/w of the total composition.

Glidants are substances that are added to a powder to improve its flowability. Examples of glidants that may be used in the pharmaceutical compositions of the present disclosure include magnesium stearate, colloidal silicon dioxide (such as the grades sold as Aerosil), starch, talc and mixtures thereof. Glidants may be present in the pharmaceutical composition at a level of from 0 to about 5% w/w.

pH adjusting agents help to maintain the stability of pharmaceutical tablet composition. For pH-modification, the addition of a base or an acid is often preferred. Examples include, but are not limited to, citric acid, fumaric acid, succinic acid, tartaric acid, malic acid, ascorbic acid, sodium bicarbonate, sodium hydroxide, sodium citrate, potassium bicarbonate, potassium hydroxide, magnesium oxide and mixtures thereof. Generally, the amount of pH adjusting agent can vary widely, for example, from about 1% to about 5% w/w of the total composition. In preferred embodiments, citric acid is used as the pH adjusting agent in the pharmaceutical tablets of the present disclosure. The pharmaceutical tablets of the present disclosure may also include one or more buffers to maintain the pH of the composition within the desired range. Examples of buffers that may be used include, but are not limited to, citrate, acetate, and the like. In preferred embodiments, the pharmaceutical tablet contains sodium citrate as a buffer in an amount sufficient to maintain the pH in the desired range of about 3.5 to about 5.5. Typically, the amount of the pH adjusting agent and the buffer used is such that it creates a pH microenvironment that is suitable for dissolution of the tablet in the mouth and absorption of buprenorphine or its pharmaceutically acceptable salt, so that the desired bioavailability of buprenorphine is achieved.

Suitable bulk sweeteners that may be used in the buprenorphine/naloxone sublingual tablets of the present disclosure include both sugar and non-sugar sweetening components. Bulk sweeteners typically constitute from about 0.01% to about 10% w/w of the total composition. Useful sweeteners are saccharide-containing components including, but not limited to, sucrose, dextrose, maltose, dextrins, trehalose, D-tagatose, dried invert sugar, fructose, levulose, galactose, corn syrup solids, and the like, alone or in combination. Sorbitol can be used as a non-sugar sweetener. Other useful non-sugar sweeteners include, but are not limited to, mannitol, xylitol, hydrogenated starch hydrolysates, maltitol, isomaltol, erythritol, lactitol and the like, alone or in combination. High-intensity artificial sweetening agents can also be used alone or in combination with the above sweeteners. Preferred high-intensity sweeteners include, but are not limited to, sucralose, aspartame, salts of acesulfame, alitame, neotame, twin sweet, saccharin and its salts, cyclamic acid and its salts, glycyrrhizin, dihydrochalcones, thaumatin, monellin, stevioside and the like, alone or in combination.

Colorants may include FD & C-type dyes and lakes, fruit and vegetable extracts, titanium dioxide, and combinations thereof. Typical flavouring agents include any natural and unnatural flavouring agents suitable for pharmaceutical applications, such as flavouring agents derived from a spice, fruit or fruit juice, vegetable or vegetable juice, and the like, for example flavours based on cocoa, lemon, caramel, vanilla, apple, apricot, berry (e.g. blackberry, red currant, black currant, strawberry, raspberry, woodberry, and the like), mint, panettone, honey, nut, malt, cola, verveine (verbena) or any combination thereof, such as for example caramel/vanilla, fruit/cream (e.g. strawberry/cream) and the like.

In one embodiment the flavouring agent of choice may be present in an amount of about 0.01 to about 5% w/w of the total composition, preferably 0.1 to 5% w/w of the total composition, most preferably 0.1 to 1% w/w of the total composition.

The pharmaceutical tablet compositions described herein are stable under various storage conditions, including refrigerated, ambient and accelerated conditions. The term "stable", as used herein, refers to physical and chemical stability of the tablet composition. The stable pharmaceutical tablet composition comprising buprenorphine and naloxone contains less than 0.5%w/w of the composition of total naloxone related degradants, and less than 1.0%w/w of the composition of total buprenorphine related degradants, when stored for at least 1 month at accelerated conditions of 40⁰C and 75% relative humidity (RH). The stable pharmaceutical tablet of the present invention contains about 0.1% w/w of the composition or less of 3-O-allyl naloxone impurity and about 0.1% w/w of the composition or less of Buprenorphine Impurity G. In one embodiment, the stable pharmaceutical tablet contains a total amount of naloxone-related degradants of about 0.2% w/w of the composition or less, and a total amount of buprenorphine-related degradants of about 0.2% w/w of the composition or less.

The stable pharmaceutical tablet composition described herein comprises the buprenorphine or pharmaceutically acceptable salt thereof in an amount equivalent to about 24 mg, naloxone or pharmaceutically acceptable salt thereof in an amount equivalent to about 6 mg and excipients selected from disintegrating agent, filler, lubricant, diluent, binder, pH adjusting agent, sweetening agent, flavoring agent, coloring agent or combinations thereof.

The stable pharmaceutical tablet composition described herein comprises the buprenorphine or pharmaceutically acceptable salt thereof in an amount equivalent to about 16 mg, naloxone or pharmaceutically acceptable salt thereof in an amount equivalent to about 4 mg and excipients selected from disintegrating agent, filler, lubricant, diluent, binder, pH adjusting agent, sweetening agent, flavoring agent, coloring agent or combinations thereof.

The stable pharmaceutical tablet composition described herein comprises the buprenorphine or pharmaceutically acceptable salt thereof in an amount equivalent to about 12 mg, naloxone or pharmaceutically acceptable salt thereof in an amount equivalent to about 3 mg and excipients selected from disintegrating agent, filler, lubricant, diluent, binder, pH adjusting agent, sweetening agent, flavoring agent, coloring agent or combinations thereof.

The stable pharmaceutical tablet composition described herein comprises the buprenorphine or pharmaceutically acceptable salt thereof in an amount equivalent to about 4 mg, naloxone or pharmaceutically acceptable salt thereof in an amount equivalent to about 1 mg and excipients selected from disintegrating agent, filler, lubricant, diluent, binder, pH adjusting agent, sweetening agent, flavoring agent, coloring agent or combinations thereof.

The stable pharmaceutical tablet composition described herein comprises the buprenorphine or pharmaceutically acceptable salt thereof, naloxone or pharmaceutically acceptable salt thereof and excipients selected from citric acid anhydrous, crospovidone, lactose monohydrate, magnesium stearate, mannitol, pregelatinized starch, povidone, sodium citrate, sucralose and flavoring agent selected from lemon, caramel, vanilla, apple.

In a particular embodiment, the process of making a stable pharmaceutical tablet for buccal or sublingual administration of the present disclosure involves admixing suitable excipients together with buprenorphine and naloxone by a variety of techniques, such as dry mixing, extrusion and/or spheronisation, or a process of granulation, which may comprise wet and/or dry granulation.

Wet granulation techniques are well known to those skilled in the art and include any technique involving the aggregation of a mix of dry primary powder particles using a granulating fluid, which fluid comprises a volatile, inert solvent, such as water, ethanol or isopropanol, either alone or in combination, and optionally in the presence of a binder or binding agent. The technique may involve forcing a wet mass through a sieve to produce pellets by spheronisation or wet granules, which are then dried. Dry granulation techniques are also well known to those skilled in the art, and include any technique in which primary powder particles are aggregated under high pressure, including slugging and roller compaction, for example, as described hereinafter. Primary particles of ingredients (for example, buprenorphine, naloxone and suitable excipients) may be processed by techniques such as grinding, dry milling, wet milling, precipitation, and the like, prior to granulation.

Granulates comprising buprenorphine and naloxone may be further processed following their formation, and prior to admixing with other ingredients, to produce a composition of the disclosure. For example, a dry granulate may be ground or milled using a suitable milling technique to produce particulate material of a smaller size, which may also be sieved to separate the desired size fraction. Wet granulate may be screened to break up agglomerates of granules and remove fine material. In either case, the unused fine material may be reworked to avoid waste. Suitable granulate particle sizes are in the range of about 0.05 mm to about 1.2 mm, such as, for example, about 0.1 mm to about 1.0 mm, about 0.2 to about 0.6 mm.

Compositions of the disclosure, once prepared, are preferably directly compressed or compacted into unit dosage forms (e.g. tablets) for administration to mammalian (e.g. human) patients.

The stable pharmaceutical tablet of the present disclosure, which are suitable for buccal or sublingual administration, are typically packaged in an inert HDPE bottles with child resistant caps (CRC) of suitable size. Alternatively, they may be packaged in child-resistant blister packs. The packaging may contain desiccants to prevent deleterious effects of moisture.

The packaged stable pharmaceutical tablets for buccal or sublingual administration possess a content uniformity that meets the requirements of the USP <905> Uniformity of Dosage Units test (incorporated herein by reference). The term "uniformity of dosage unit" is defined as the degree of uniformity in the amount of the drug substance among dosage units. The test for "Content Uniformity" of compositions presented in dosage units is based on the assay of the individual content of the drug substance(s) in a number of dosage units to determine whether the individual content is within the limits set. It is crucial that the assay and content uniformity meet the specifications, since there is a relatively low concentration of the active ingredients, i.e., buprenorphine, and especially naloxone, in the tablets, to ensure they contain accurate and uniform amount of the drug substances during routine commercial manufacturing and storage. Through wet granulation, blending, and compression processes, optimal assay and content uniformity was achieved.

In some embodiments, the assay of buprenorphine and naloxone in the packaged stable pharmaceutical tablet for buccal or sublingual administration of the present disclosure is in the range of about 85% w/w to about 115% w/w of the product label claim, as determined by USP method for the finished product, and/or as compared to other buprenorphine/naloxone dosage forms that may be available on the market (for example Suboxone^{-̅} or equivalents thereof).

In another embodiment, the rate of disintegration (disintegration time) of the stable pharmaceutical tablet for buccal or sublingual administration of the present disclosure in the mouth of the patient is about 20 minutes or less, preferably about 15 minutes or less. The rate of disintegration of the compositions of the present disclosure can be measured using various in vitro and in vivo test methods, for example the USP <701> disintegration test.

The stable pharmaceutical tablet composition described herein has an in vitro disintegration time of less than 120 seconds as per the USP test, hardness of about 2 to about 10kP, friability of less than about 1%, in vivo disintegration of less than 20 minutes, and releases more than 85% of the buprenorphine in less than about 5 minutes, and all of the naloxone in less than about 20 minutes. The dissolution is carried out in a dissolution apparatus as defined in the USP, and known to a skilled person.

The stable pharmaceutical tablet composition described herein has in vivo disintegration time of less than about 20 minutes, more preferably less than about 15 minutes, still more preferably less than about 10 minutes upon prior hydration with 200 mL of water, 1 hour prior to the study. Such a study is detailed in Example below.

### Definitions

Unless otherwise defined, all technical and scientific terms used herein have the same meaning as commonly understood by one of ordinary skill in the art to which this disclosure belongs. The terminology used in the description of the disclosure herein is for the purpose of describing particular embodiments only, and is not intended to be limiting of the disclosure.

Unless the context indicates otherwise, it is specifically intended that the various features of the disclosure described herein can be used in any combination.

Moreover, the present disclosure also contemplates that in some embodiments of the disclosure, any feature or combination of features set forth herein can be excluded or omitted.

All publications, patent applications, patents, and other references mentioned herein are incorporated by reference herein in their entirety for all purposes.

As used herein, "a," "an," or "the" can mean one or more than one. Also, as used herein, "and/or" refers to and encompasses any and all possible combinations of one or more of the associated listed items, as well as the lack of combinations when interpreted in the alternative ("or").

Furthermore, the term "about," as used herein when referring to a measurable value such as an amount of a compound or agent of this disclosure, dose, time, temperature, and the like, is meant to encompass variations of ±10% of the specified amount.

As used herein, the term "buprenorphine" includes buprenorphine base and its pharmaceutically acceptable salts, solvates, hydrates and polymorphs thereof. Similarly, the term "naloxone", as used herein, includes naloxone base and its pharmaceutically acceptable salts, solvates, hydrates and polymorphs thereof.

The terms composition, formulation, tablet, pharmaceutical tablet, pharmaceutical composition, dosage form(s) are used interchangeably.

The term dosage form(s) as used herein includes solid dosage forms such as tablets, capsules, pellets, granules and the like.

A unit dosage form refers to a specific quantity of a medication or pharmaceutical product that is packaged as a single dose, intended for administration to a patient.

The present disclosure is further illustrated by reference to the following examples which is for illustrative purposes only, and does not limit the scope of the disclosure in any manner.

### Examples

### Example 1

**Table 1**

| **Ingredients** | **%w/w** | **mg/tablet 16/4mg** | **mg/tablet 12/3mg** | **mg/tablet 4/1mg** |
|---|---|---|---|---|
| Buprenorphine HCl, USP | 4.33 | 17.3 | 12.975 | 4.325 |
| Naloxone HCl dihydrate USP | 1.23 | 4.9 | 3.675 | 1.225 |
| Lactose monohydrate 312, NF | 36.78 | 147.1 | 110.325 | 36.775 |
| Mannitol (Pearlitol SD 200) USP | 37.68 | 150.7 | 113.025 | 37.675 |
| Povidone (Kollidon K 30), USP | 1.00 | 4 | 3 | 1 |
| Pregelatinized starch, USP | 3.75 | 15 | 11.25 | 3.75 |
| Sucralose, NF | 0.75 | 3 | 2.25 | 0.75 |
| N-Lemon flavor | 0.75 | 3 | 2.25 | 0.75 |
| Citric acid anhydrous, USP | 3.0 | 12 | 9 | 3 |
| Sodium citrate anhydrous, USP | 3.0 | 12 | 9 | 3 |
| Crospovidone (Polyplasdone XL-10), Intragranular USP | 3.0 | 12 | 9 | 3 |
| Crospovidone (Polyplasdone XL-10), Intragranular USP | 3.5 | 14 | 10.5 | 3.5 |
| Magnesium stearate | 1.25 | 5 | 3.75 | 1.25 |
| Purified water (removed during | 9.00 | 36 | 27 | 9 |
| drying) | | | | |
| **Total** | **100.0** | **400** | **300** | **100** |

The buccal/sublingual composition was obtained by the following manufacturing process - All of the ingredients were weighed according to the details provided in Table 1 above. The dispensed materials were screened using the US standard sieve #30. Citric acid, sodium citrate and naloxone HCl were directly dissolved in water making a clear binding solution. Buprenorphine HCl was sandwiched in between all other excipients for uniform distribution.

**Granulation Dry mix:** A conventional high shear wet granulator was used for granulation. The screened excipients were transferred into the granulator bowl and subjected to dry mixing for 3 minutes, using the impeller at a speed of 200 rpm. The binding solution was prepared by adding citric acid, sodium citrate and naloxone HCl in the weighed quantity of water until the ingredients became clear.

**Wet granulation:** After dry mixing, the binding solution was slowly poured into the granulator bowl within 1 minute, and mixed at an impeller speed of 200 rpm and chopper speed of 1000 rpm. Water rinsing was immediately initiated with impeller and chopper speeds set to 200 rpm and 1000 rpm, respectively. After rinsing, the wet granules were subjected to wet massing for 1 minute with the impeller and chopper speeds set to 200 rpm and 1000 rpm, respectively.

**Drying:** The wet granules were screened through a 14 mesh hand screen, to control the granule size and ensure uniform drying. Granules were spread onto trays and subjected to oven drying at 45°C to 55°C until LOD was NMT 2%.

**Screening:** The dried granules were then subjected to milling using a Fitzmill in which the final weight of the granules was determined.

**Blending:** The weight of the crospovidone was adjusted based on the screened granules. The screened granules and crospovidone were added into a V-blender and pre-blended for 5 minutes. The magnesium stearate level was also adjusted based on the final weight of the granules, screened through a 30 mesh hand screen, and then added into the blender and blended for 3 minutes.

**Compression:** The final blend was compressed into tablets of three strengths (16/4, 12/3 & 4/1 mg).

### Example 2

The formulated tablet composition of Example 1 was further characterised for physical parameters. Table 2 mentions the physical parameters of the tablet composition thus analysed.

**Table 2**

| **Description** | **Tablet of example 1** | | |
|---|---|---|---|
| **Strength** | **16/4 mg** | **12/3 mg** | **4/1 mg** |
| **Tablet weight** | 400 mg | 300 mg | 100 mg |
| **Score** | Yes | Yes | Yes |
| **Shape** | Round | Round | Round |
| **Size (diameter ± 1 mm)** | 10mm | 9 mm | 6 mm |
| **USP Disintegration Time (mins)** | < 2 minutes 60-120 seconds | < 2 minutes 60-120 seconds | < 2 minutes 60-120 seconds |
| **Hardness (kp)** | 2-7 kp | 2-7 kp | 1-6 kp |
| **Thickness** | 4 mm- 5 mm | 4 mm- 5 mm | 4 mm- 5 mm |
| **Friability** | < 1 % | < 1 % | < 1 % |
| **Packaging** | HDPE Bottles with Dunnage (Rayon) and Desiccant | | |

### Example 3

The following dissolution method, recommended by the USFDA for buprenorphine/naloxone sublingual tablets, was used to test the dissolution profiles of the tablets of Example 1.

**Table 3**

| **Drug Name** | **Dosage Form** | **USP Apparatus** | **Speed (RPM)** | **Medium** | **Volume (mL)** | **Recommended Sampling Times (minutes)** |
|---|---|---|---|---|---|---|
| Buprenorphine HCl/ Naloxone HCl | Tablet (Sublingual ) | I (Basket) | 100 | Water | 500 | 1, 3, 5, 7.5, 10, 15 and 20 |

The following Table 4 provides the comparative dissolution profiles of the tablets of Example 1 versus the reference product, which is a buprenorphine/naloxone tablet approved as a generic to Suboxone^{-̅} (Hikma Lot # AB9292A), the dissolution being conducted using the dissolution conditions of Table 3. As seen from the data in Table 4, due to high solubility of the active ingredients, as well as relatively faster disintegration times, the dissolution rates are very fast.

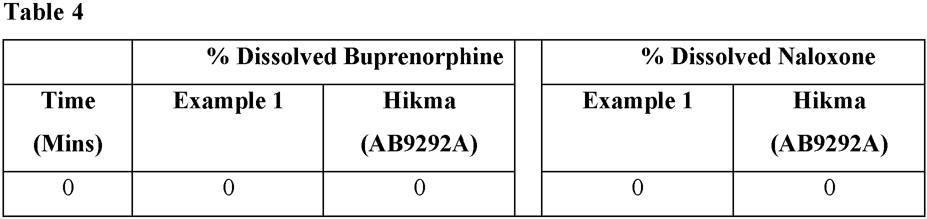

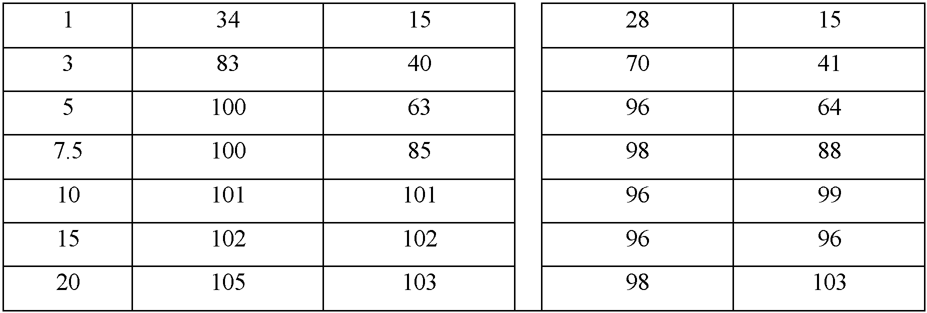

### Example 4

Buprenorphine/Naloxone Tablets, 16/4 mg (Example 1) were studied for stability under accelerated conditions of 40⁰ C/75 % RH (ACC). Table 5 summarizes the data and shows that the product is stable. Furthermore, the product did not show any difference with and without nitrogen purge in the bottles during packaging. All known and unknown impurities and degradants were found to be below quantitation limits at 1M under accelerated conditions. Furthermore, the assay and pH of the tablets also was seen to be stable.

**Table 5**

| **Impurity** | **Example 1** | | **Example 1 (With Nitrogen Purge)** |
|---|---|---|---|
| | **T0** | **1M ACC** | **1M ACC** |
| **Naloxone impurities** | | | |
| Noroxymorphone (Naloxone Impurity A) | <0.1 | <0.1 | <0.1 |
| 3-O Allyl Naloxone (Naloxone Impurity B) | <0.1 | <0.1 | <0.1 |
| 10 alpha hydroxy naloxone (Naloxone | <0.1 | <0.1 | <0.1 |
| Impurity C) | | | |
| 10 beta hydroxy naloxone (Naloxone Impurity F) | <0.1 | <0.1 | <0.1 |
| Naloxone Dimer | <0.1 | <0.1 | <0.1 |
| Total Naloxone related degradants | <0.1 | <0.1 | <0.1 |
| Naloxone Assay | 98.5 % | 99.4 % | 98.4 % |

| **Buprenorphine impurities** | | | |
|---|---|---|---|
| Norbuprenorphine (Buprenorphine Impurity B) | <0.1 | <0.1 | <0.1 |
| Buprenorphine Impurity E | <0.1 | <0.1 | <0.1 |
| Buprenorphine Impurity F | <0.1 | <0.1 | <0.1 |
| Buprenorphine Impurity G | <0.1 | <0.1 | <0.1 |
| Buprenorphine Impurity I | <0.1 | <0.1 | <0.1 |
| Total Buprenorphine related degradants | 0.2 | 0.2 | 0.2 |
| Buprenorphine Assay | 100.9 % | 102.5 % | 101.4 % |
| pH | 4.2 | 4.3 | 4.3 |

The IUPAC names and structures of the impurities listed in Table 5 above have been included in Table 6 below -

**Table 6**

| **Impurity** | **IUPAC name** | **Chemical structure** |
|---|---|---|
| **Naloxone impurities** | | |
| Noroxymorphone (Naloxone Impurity A) | 4,5α-Epoxy-3,14-dihydroxymorphinan-6-one | 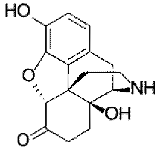 |
| 3-O Allyl Naloxone (Naloxone Impurity B) | 4,5α-Epoxy-14-hydroxy-17-(prop-2-enyl)-3-(prop-2-enyloxy)morphinan-6-one | 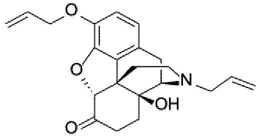 |
| 10 alpha hydroxy naloxone (Naloxone Impurity C) | 4,5α-Epoxy-3,10α,14- trihydroxy-17-(prop-2-enyl) morphinan-6-one | 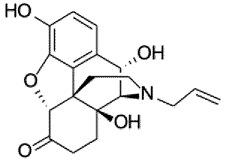 |
| 7,8-Didehydronaloxone (Naloxone Impurity D) | 7,8-Didehydro-4,5α-epoxy-3,14-dihydroxy-17-(prop-2-enyl)morphinan-6-one | 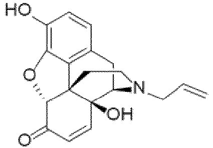 |
| 2,2'-Binaloxone (Naloxone Impurity E) | 4,5α:4',5'α-Diepoxy-3,3',14,14'-tetrahydroxy-17,17'-bis(prop-2-enyl)-2,2'-bimorphinanyl-6,6'-dione | 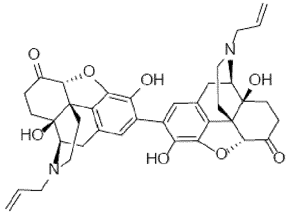 |
| 10 beta hydroxy naloxone (Naloxone Impurity F) | 4,5α-Epoxy-3,10β,14-trihydroxy-17-(prop-2-enyl)morphinan-6-one | 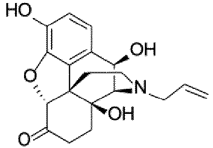 |
| Naloxone Dimer (2,2'-Bisnaloxone) | 4,5α:4',5'α-diepoxy-3,3',14,14'-tetrahydroxy-17,17'-bis(prop-2- enyl)-2,2'- | 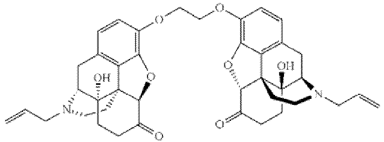 |
| | bimorphinanyl-6,6'-dione | |

| **Buprenorphine related impurities** | | |
|---|---|---|
| **Impurity** | **IUPAC name** | **Chemical structure** |
| N-But-3-enyl norbuprenorphine (Buprenorphine Impurity A) | (2S)-2-[17-(but-3-enyl)-4,5α-epoxy-3-hydroxy-6-methoxy- 6α,14-ethano-14α-morphinan-7α- yl]-3,3-dimethylbutan-2-ol | 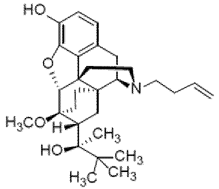 |
| Norbuprenorphine (Buprenorphine Impurity B) | (2S)-2-(4,5α-epoxy-3-hydroxy-6-methoxy-6α,14- ethano-14α-morphinan-7α-yl)-3,3-dimethylbutan-2-ol | 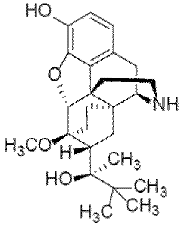 |
| 3-O-Methyl-N-cyano-norbuprenorphin e (Buprenorphine Impurity C) | 4,5α-epoxy-7α-[(1*S*)-1-hydroxy-1,2,2-trimethylpropyl]-3,6-dimethoxy-6α,14-ethano-14α- morphinan-17-carbonitrile | 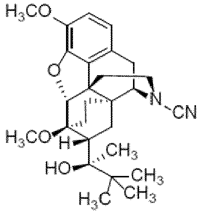 |
| 3-O-Methylbuprenorphine (Buprenorphine Impurity D) | (2*S*)-2-[17-(cyclopropylmethyl)- 4,5α-epoxy-3,6-dimethoxy-6α,14- ethano-14α-morphinan-7α-yl]- 3,3-dimethylbutan-2-ol | 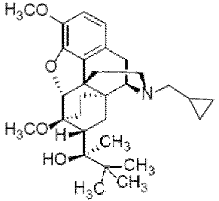 |
| Buprenorphine Impurity E | (2*S*)-2-[17-(cyclopropylmethyl)-4,5α- epoxy-3,6-dihydroxy- 6α,14-ethano-14α-morphinan-7α-yl]-3,3-dimethylbutan-2-ol | 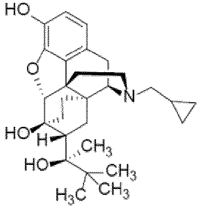 |
| Buprenorphine Impurity F | 17-(cyclopropylmethyl)-4,5α- epoxy-6-methoxy-7α-[1-(1,1-dimethylethyl)enthenyl]-6α,14- ethano-14α-morphinan-3-ol | 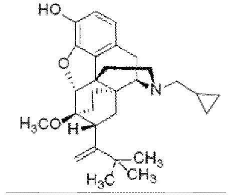 |
| Buprenorphine Impurity G | 17,17'-di(cyclopropylmethyl)-4,5α;4',5α'-diepoxy-7α,7α'-di [(1S)-1-hydroxy-1,2,2-trimethylpropyl]-6, 6'-dimethoxy-2,2'-bi(6α, 14-ethano-14α-morphinan)-3,3'-diol | 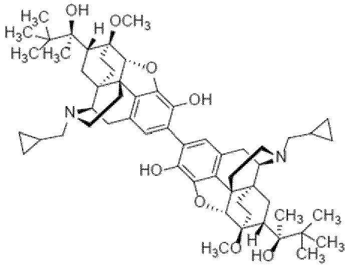 |
| Buprenorphine Impurity I | 8α,12α-Ethano-4,8-methano- 12βH-Benzofuro [3,2e]furo[2,3-g]isoquinolin-1- ol,7-(cyclopropylmethyl)-5,6,7,8,9,9α,10,11-octahydro- 10,10,11,11-tetramethyl (4βS,8R,8αS,9αR, 12αR, 1 2βR) | 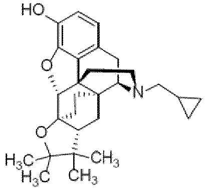 |

### Example 5

In vivo disintegration times were conducted on two formulations with different in vitro disintegration rates. The purpose of the study was also to understand the effect of disintegration time of 2 tablets under the tongue vs 1 tablet.

Two batches were prepared based on the process detailed in example 1 (batch# 1027-15C and 1027-10B). These tablets were tested for disintegration under the tongue in 6 subjects. One tablet of batch #1027-15 C was used, while 2 tablets (one on each side under the tongue) of batch #1027-10B were used. The invitro Disintegration Time (DT) for the two batches, using USP disintegration apparatus showed that the DT of batch #1027-15 C was longer than that for batch #1027-10B.

Furthermore, the effect of hydration was also studied. This was accomplished by studying the effect of disintegration time with the subjects hydrated with 200 mL of water 1 hour prior to the study vs patients administered the tablets without any hydration of the mouth prior to administration. For both studies (with and without hydration) subjects were told to use 60 mL of water to rinse their mouths and spit the water prior to administering the tablets.

Table 7 below summarizes the data. As seen, the in vivo disintegration of batch #1027-15 C (1 tablet) was similar to that of batch #1027-10 B (2 tablets) in both hydrated as well as non- hydrated conditions.

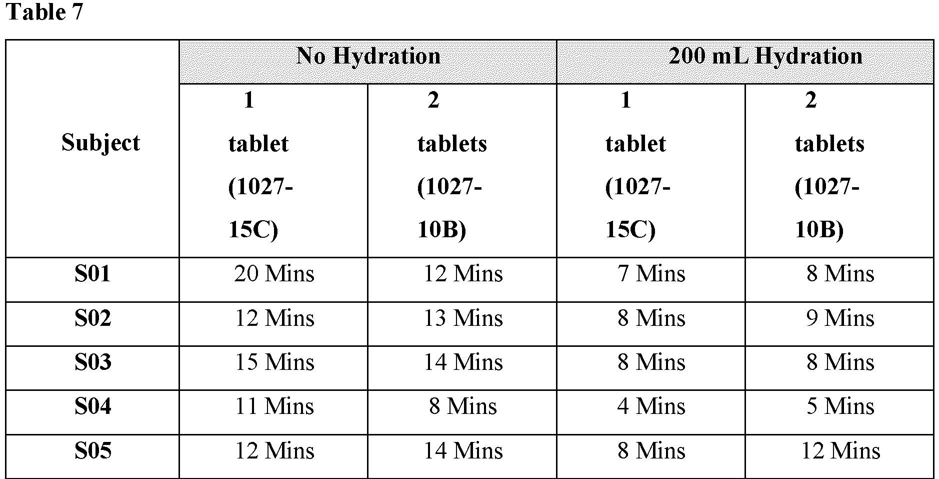

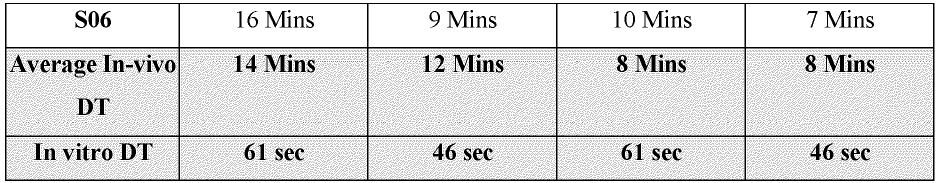

### Example 6

### Single Dose Comparative Bioavailability of Study of Buprenorphine 16 mg /Naloxone 4 mg Sublingual Tablets vs. Buprenorphine 8 mg /Naloxone 2 mg Sublingual Tablets (2 X 8 mg /2 mg) in Healthy Volunteers

An open label, randomized, two treatment, two sequence, four period, fully replicate, single dose, cross over, bioequivalence study was conducted to assess the comparative bioavailability of Buprenorphine 16 mg /Naloxone 4 mg Sublingual Tablets (Example 1; Batch # D1027-58) and Buprenorphine 8 mg /Naloxone 2 mg sublingual tablets of Hikma (Lot # AC0624A) in 12 healthy volunteers.

Subjects were randomized to receive the following two treatments, with a washout period of 10 days between each treatment:
- **Treatment A:** Buprenorphine/Naloxone (16 mg/4 mg) sublingual tablets, administered as a single dose under fasting conditions.
- **Treatment B:** Buprenorphine/Naloxone (8 mg/2 mg) sublingual tablets (2 x 8 mg/2 mg), administered as a single dose under fasting conditions.

**Dosing Schedule:** After screening and baseline testing, subjects received either the Test or Reference formulation in each study period (4 periods total). Following each dosing day, a washout period of 10 days was observed. This pattern was maintained for all four study periods.

**Pharmacokinetic Assessment:** Blood samples were collected at designated time points over a 72-hour period post-dose in each period to evaluate pharmacokinetic parameters, including Cmax, AUCO-∞, Tmax, t½, Kel, and AUC_%Extrap_Obs.

**Bioequivalence Criteria:** The bioequivalence of the Test product to the Reference standard was evaluated based on the 90% confidence interval for the ratios of Test/Reference for the parameters Cmax, AUC0-t, and AUCO-∞, with acceptance criteria set between 80% and 125%.

### Results:

| **Parameter** | **Treatment A (Test)** | **Treatment B (Reference)** | **90% CI for T/R Ratio (%)** | **Conclusion** |
|---|---|---|---|---|
| Cmax (Buprenorphine) | 11636.42 pg/mL | 10512.97 pg/mL | 97.98 - 125.05 | Bioequivalent |
| AUC0-t (Buprenorphine) | 72954.07 pg·h/mL | 63195.66 pg·h/mL | 107.76 - 123.67 | Bioequivalent |
| AUCO-∞ (Buprenorphine) | 80364.87 pg·h/mL | 71105.91 pg·h/mL | 106.00 - 120.50 | Bioequivalent |
| Cmax (Naloxone) | 457.44 ng/mL | 442.40 ng/mL | 88.34 - 121.02 | Bioequivalent |
| AUC0-t (Naloxone) | 1031.11 ng·h/mL | 939.87 ng·h/mL | 97.99 - 122.82 | Bioequivalent |
| AUCO-∞ (Naloxone) | 1089.71 ng·h/mL | 990.70 ng·h/mL | 98.19 - 123.22 | Bioequivalent |

| | | | | |
|---|---|---|---|---|
| **Conclusion:** The pharmacokinetic analysis confirmed bioequivalence between the test (16 mg/4 mg) and reference (8 mg/2 mg) sublingual formulations, meeting bioequivalence criteria for both buprenorphine and naloxone. | | | | |

## Claims

1. A stable pharmaceutical tablet for buccal or sublingual administration, comprising:
- from about 1 mg to about 24 mg of buprenorphine in the form of its base or a pharmaceutically acceptable salt; and
- from about 0.5 mg to about 6 mg of naloxone in the form of its base or a pharmaceutically acceptable salt; and pharmaceutically acceptable excipients,
wherein the tablet:
- has an in vitro disintegration time of less than 120 seconds when tested by the disintegration test defined in the United States Pharmacopoeia (USP);
- has a hardness of 2-10 kP;
- has a friability of less than 1%;
- exhibits an in vivo disintegration time of less than 20 minutes; and
- releases more than 85% of the buprenorphine in less than 5 minutes and all of the naloxone in less than 20 minutes, when tested in vitro in a dissolution apparatus defined in the United States Pharmacopoeia (USP).

2. The pharmaceutical tablet according to claim 1, wherein the amount of buprenorphine or pharmaceutically acceptable salt thereof is about 16 mg and the amount of naloxone or its pharmaceutically salt is about 4 mg.

3. The pharmaceutical tablet according to claim 1, wherein the amount of buprenorphine or pharmaceutically acceptable salt thereof, in the tablet is about 12 mg and the amount of naloxone or its pharmaceutically salt is about 3 mg.

4. The pharmaceutical tablet according to claim 1, wherein the amount of buprenorphine or pharmaceutically acceptable salt thereof, in the tablet is about 4 mg and the amount of naloxone or its pharmaceutically salt is about 1 mg.

5. The pharmaceutical tablet according to claim 1, wherein the amount of buprenorphine or pharmaceutically acceptable salt thereof, in the tablet is about 24 mg and the amount of naloxone or its pharmaceutically salt is about 6 mg.

6. The pharmaceutical tablet according to claim 1, wherein the in vivo disintegration time is less than 20 minutes when the tablet is administered without any pre-hydration of the mouth with water.

7. The pharmaceutical tablet according to claim 1, wherein the in vivo disintegration time is less than 10 minutes when the tablet is administered with pre-hydration of the mouth with water.

8. The pharmaceutical tablet according to claim 1, further comprising a score mark or separation mark to guide a user in breaking the tablet along the designated area or region indicated by said score or separation mark.

9. The pharmaceutical tablet according to claim 1, further comprising pharmaceutically acceptable excipients selected from one or more of disintegrating agent, filler, lubricant, diluent, binder, pH adjusting agent, buffer, sweetening agent, flavouring agent, and a combination thereof.

10. The pharmaceutical tablet according to claim 9, wherein the pH adjusting agent is citric acid and the buffer is sodium citrate.

11. The stable pharmaceutical tablet according to claim 10, wherein upon storage under accelerated stability conditions of 40°C and 75% relative humidity for at least one month, the tablet contains about 0.1%w/w of the tablet or less of 3-O-allyl naloxone impurity and about 0.1% or less of Buprenorphine Impurity G.

12. The stable pharmaceutical tablet according to claim 11, further comprising a total amount of naloxone-related degradants of about 0.2%w/w of the tablet or less and a total amount of buprenorphine-related degradants of about 0.2%w/w of the tablet or less.
